(19) Europäisches Patentamt
European Patent Office
Office européen des brevets

(11) Veröffentlichungsnummer: **0 040 367**
**A1**

(12) # EUROPÄISCHE PATENTANMELDUNG

(21) Anmeldenummer: **81103478.4**

(22) Anmeldetag: **07.05.81**

(51) Int. Cl.³: **C 07 D 249/08**
A 01 N 43/64

(30) Priorität: **19.05.80 DE 3019045**

(43) Veröffentlichungstag der Anmeldung:
**25.11.81 Patentblatt 81/47**

(84) Benannte Vertragsstaaten:
**AT BE CH DE FR GB IT LI NL SE**

(71) Anmelder: **BAYER AG**
**Zentralbereich Patente, Marken und Lizenzen**
**D-5090 Leverkusen 1, Bayerwerk(DE)**

(72) Erfinder: **Reiser, Wolf, Dr.**
**Kiebitzweg 17**
**D-5600 Wuppertal 1(DE)**

(72) Erfinder: **Elbe, Hans-Ludwig, Dr.**
**Dasnoeckel 59**
**D-5600 Wuppertal 11(DE)**

(72) Erfinder: **Büchel, Karl Heinz, Prof. Dr.**
**Dabringhausener Strasse 42**
**D-5063 Burscheid(DE)**

(72) Erfinder: **Frohberger, Paul-Ernst, Dr.**
**Willi-Baumeister-Strasse 5**
**D-5090 Leverkusen(DE)**

(72) Erfinder: **Brandes, Wilhelm, Dr.**
**Eichendorffstrasse 3**
**D-5653 Leichlingen(DE)**

(54) **Substituierte Phenyl-triazolyl-vinyl-ketone, Verfahren zu ihrer Herstellung sowie ihre Verwendung als Fungizide.**

(57) Substituierte Phenyl-triazolyl-vinyl-ketone der allgemeinen Formel

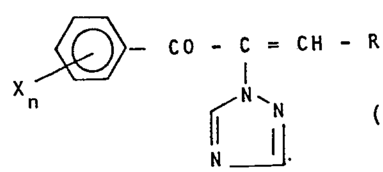

(1)

in welcher

R für Methyl, Ethyl, Isopropyl, Alkyl mit mehr als 3 Kohlenstoffatomen, gegebenenfalls substituiertes Cycloalkyl und Cycloalkenyl, gegebenenfalls substituiertes Alkenyl und Alkinyl, gegebenenfalls substituiertes Phenoxyphenyl sowie gegebenenfalls im Alkylteil und im Phenylteil substituiertes Phenylalkyl steht,

X für Halogen, gegebenenfalls substituiertes Phenyl und Phenoxy steht und

n für die Zahlen 0, 1, 2 und 3 steht,

und deren physiologisch verträglichen Säureadditions-Salze sowie Metallsalz-Komplexe, werden erhalten, wenn man zum Beispiel Ketoenamine mit magnesium-organischen Verbindungen in Gegenwart eines Lösungsmittels und gegebenenfalls in Gegenwart eines Katalysators umsetzt; daneben gibt es noch andere Verfahrensvarianten.

Als Pflanzenschutzmittel Können die erfindungsgemäß verwendbarer. Wirkstoffe in besonders gutem Erfolg zur Bekämpfung solcher Pilze eingesetzt werden, die echte Mehltauerkrankungen hervorrufen.

EP 0 040 367 A1

BAYER AKTIENGESELLSCHAFT
Zentralbereich
Patente,Marken und Lizenzen

5090 Leverkusen, Bayerwerk
Slr/W
I b

Substituierte Phenyl-triazolyl-vinyl-ketone, Verfahren zu ihrer Herstellung sowie ihre Verwendung als Fungizide

Die vorliegende Erfindung betrifft neue substituierte Phenyl-triazolyl-vinyl-ketone, mehrere Verfahren zu ihrer Herstellung sowie ihre Verwendung als Fungizide.

Es ist bereits bekannt geworden, daß substituierte Phenacyl-triazolyl-styrol-Derivate, wie insbesondere 1-(2,4-Dichlorphenacyl)-1-(1,2,4-triazol-1-yl)-styrol-Derivate, gute fungizide Wirksamkeit besitzen (vergleiche DE-OS 26 45 617). Die Wirkung dieser Verbindungen ist jedoch, insbesondere bei niedrigen Aufwandmengen und -konzentrationen, nicht immer ganz befriedigend.

Es wurden neue substituierte Phenyl-triazolyl-vinyl-ketone der allgemeinen Formel

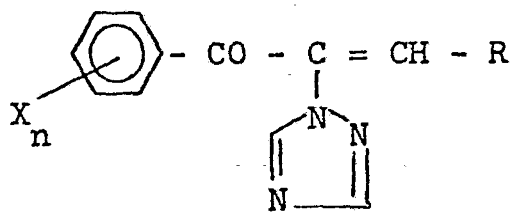

(I)

in welcher

R für Methyl, Ethyl, Isopropyl, Alkyl mit mehr als 3 Kohlenstoffatomen, gegebenenfalls substituiertes Cycloalkyl und Cycloalkenyl, gegebenenfalls substituiertes Alkenyl und Alkinyl, gegebenenfalls substituiertes Phenoxyphenyl sowie gegebenenfalls im Alkylteil und im Phenylteil substituiertes Phenylalkyl steht,

X für Halogen, gegebenenfalls substituiertes Phenyl und Phenoxy steht und

n für die Zahlen 0,1,2 und 3 steht,

und deren physiologisch verträglichen Säureadditions-Salze sowie Metallsalz-Komplexe gefunden.

Die Verbindungen der Formel (I) können in zwei geometrischen Isomerenformen (E- und Z-Form) vorliegen, je nach Anordnung der Gruppen, die an die Doppelbindung gebunden sind; vorzugsweise fallen sie in einem wechselnden E,Z-Isomerenverhältnis an. Die vorliegende Erfindung betrifft sowohl die einzelnen Isomeren als auch die Isomeren-Gemische.

0040367

Weiterhin wurde gefunden, daß man die substituierten Phenyl-triazolyl-vinyl-ketone der Formel (I) erhält, wenn man

a) Ketoenamine der Formel

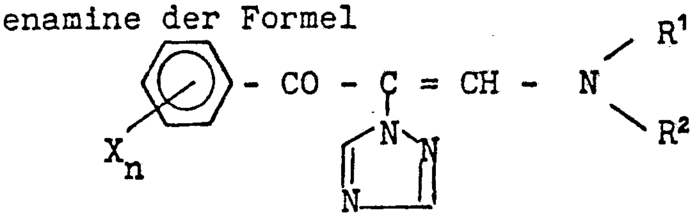

(II)

in welcher

X und n die oben angegebene Bedeutung haben und

$R^1$ und $R^2$ gleich oder verschieden sind und und für Alkyl stehen,

mit magnesium-organischen Verbindungen der Formel

Hal - Mg - R (III)

in welcher

R die oben angegebene Bedeutung hat und

Hal für Halogen steht,

in Gegenwart eines Lösungsmittels und gegebenenfalls in Gegenwart eines Katalysators umsetzt, oder

b) Triazolyl-ketone der Formel

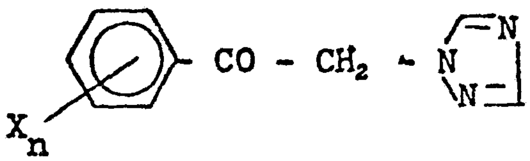 (IV)

in welcher

X und n die oben angegebene Bedeutung haben,

mit Aldehyden der Formel

$$O = CH - R \qquad (V)$$

in welcher

R die oben angegebene Bedeutung hat,

in Gegenwart eines Lösungsmittels und in Gegenwart eines Katalysators umsetzt.

An die so erhaltenen Verbindungen der Formel (I) kann gegebenenfalls eine Säure oder ein Metallsalz addiert werden.

Die neuen substituierten Phenyl-triazolyl-vinyl-ketone der Formel (I) weisen starke fungizide Eigenschaften auf. Dabei zeigen überraschenderweise die erfindungsgemäßen Verbindungen eine bessere fungizide Wirksamkeit als die aus dem Stand der Technik bekannten 1-(2,4-Dichlorphenacyl)-1-(1,2,4-triazol-1-yl)-styrol-Derivate, welche chemisch und wirkungsmäßig naheliegende Verbindungen sind. Die erfindungsgemäßen Stoffe stellen somit eine Bereicherung der Technik dar.

Die erfindungsgemäßen substituierten Phenyl-triazolyl-vinyl-ketone sind durch die Formel (I) allgemein definiert. In dieser Formel steht R vorzugsweise für Methyl, Ethyl, Isopropyl sowie für geradkettiges oder verzweigtes Alkyl mit 4 bis 29, insbesondere bis 18, Kohlenstoffatomen; für gegebenenfalls durch Alkyl mit 1 bis 4 Kohlenstoffatomen substituiertes Cycloalkyl und Cycloalkenyl mit jeweils 5 bis 7 Kohlenstoffatomen; für gegebenenfalls substituiertes, geradkettiges oder verzweigtes Alkenyl und Alkinyl mit jeweils bis zu 6 Kohlenstoffatomen, wobei als Substituenten genannt seien: Hydroxy, Alkoxy mit 1 bis 4 Kohlenstoffatomen sowie Phenyl, das gegebenenfalls substituiert sein kann durch Halogen und Alkyl mit 1 bis 4 Kohlenstoffatomen; für gegebenenfalls substituiertes Phenoxyphenyl mit Halogen und Alkyl mit 1 bis 4 Kohlenstoffatomen als Substituenten; sowie für gegebenenfalls substituiertes Phenalkyl mit 1 bis 4 Kohlenstoffatomen im Alkylteil, wie insbesondere Benzyl, wobei als Phenylsubstituenten vorzugsweise infrage kommen: Halogen, Alkyl mit 1 bis 4 Kohlenstoffatomen sowie Halogenalkyl mit 1 bis 4 Kohlenstoffatomen und 1 bis 5 gleichen oder verschiedenen Halogenatomen, wie insbesondere Fluor- und Chloratomen, und wobei als Alkylsubstituenten vorzugsweise infrage kommen: Cyano, Hydroxycarbonyl und Alkoxycarbonyl mit 1 bis 4 Kohlenstoffatomen im Alkylteil.

X steht vorzugsweise für Halogen sowie für gegebenenfalls substituiertes Phenyl und Phenoxy, wobei als Substituenten vorzugsweise infrage kommen: Halogen, Alkyl mit 1 bis 4 Kohlenstoffatomen und Halogenalkyl mit 1 bis 4 Kohlenstoffatomen und 1 bis 5 gleichen oder verschiedenen Halogenatomen, wie insbesondere Fluor- und Chloratomen.

Der Index n hat vorzugsweise die in der Erfindungsdefinition angegebene Bedeutung.

Besonders bevorzugt sind diejenigen Verbindungen der Formel (I) in denen R für gegebenenfalls durch Methyl und Ethyl substituiertes Cyclohexyl, Cyclohexenyl, Cyclopentyl oder Cyclopentenyl steht; für Methacryl, Trimethylvinyl, Acetylenyl, Hydroxyacetylenyl, Methoxyacetylenyl, Phenacetylenyl, Chlorphenylacetylenyl, Propargyl, Hydroxypropargyl, Methoxypropargyl, Phenylpropargyl oder Chlorphenylpropargyl steht; für gegebenenfalls im Phenylteil durch Fluor, Chlor und Methyl substituiertes Benzyl steht, wobei die Methylengruppe außerdem substituiert sein kann durch Cyano, Hydroxycarbonyl oder Methoxycarbonyl; sowie für gegebenenfalls durch Fluor, Chlor oder Methyl substituiertes Phenoxyphenyl steht; X für Fluor, Chlor oder Brom steht; sowie für gegebenenfalls durch Fluor, Chlor, Brom, Methyl, Ethyl, Isopropyl oder tert.-Butyl substituiertes Phenyl oder Phenoxy steht; und der Index n für die Zahlen 0,1 oder 2 steht.

Verwendet man beispielsweise 1-(2,4-Dichlorphenyl)-3-dimethylamino-2-(1,2,4-triazol-1-yl)-2-propen-1-on und Phenylacetylen-magnesiumbromid als Ausgangsstoffe, so kann der Reaktionsablauf durch das folgende Formelschema wiedergegeben werden (Verfahren a):

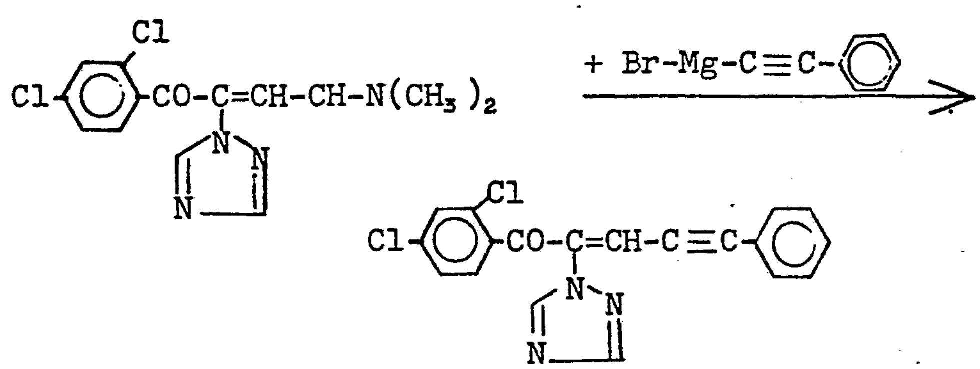

Verwendet man beispielsweise α-(1,2,4- Triazol-1-yl)-2,4-dichloracetophenon und Cyclohexancarbaldehyd als Ausgangsstoffe, so kann der Reaktionsablauf durch das folgende Formelschema wiedergegeben werden (Verfahren b):

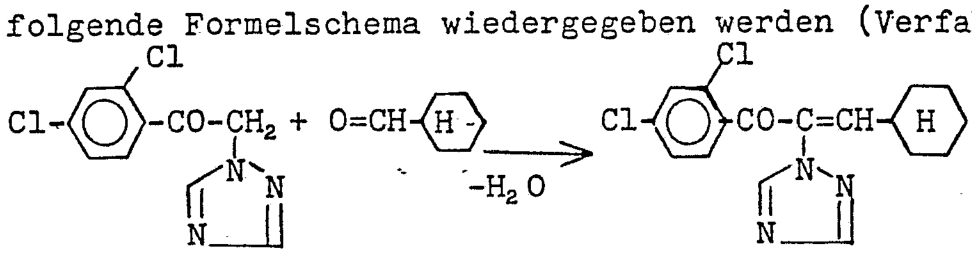


Die als Ausgangsstoffe für das erfindungsgemäße Verfahren (a) zu verwendenden Ketoenamine sind durch die Formel (II) allgemein definiert. In dieser Formel stehen X und n vorzugsweise für diejenigen Reste, die bereits im Zusammenhang mit der Beschreibung der erfindungsgemäßen Stoffe der Formel (I) vorzugsweise für diese Substituenten genannt wurden. $R^1$ und $R^2$ sind gleich oder verschieden und stehen vorzugsweise für Alkyl mit 1 bis 4 Kohlenstoffatomen, insbesondere für Methyl.

Die Ketoenamine der Formel (II) sind noch nicht bekannt; sie sind jedoch Gegenstand einer eigenen älteren Anmeldung, die noch nicht veröffentlicht ist (vergleiche die deutsche Patentanmeldung P 30 00 643.0 vom 10.1.80 [Le A 20 084]). Die Ketoenamine der Formel (I) können nach den dort beschriebenen Verfahren erhalten werden, indem man Triazolyl-ketone der Formel (IV) mit Amidacetalen bzw. Aminalestern der Formeln

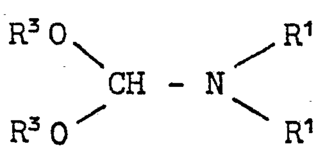
(VI a)

bzw.

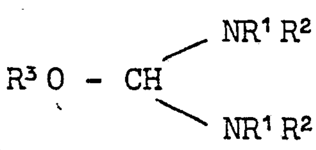
(VI b)

worin $R^1$ und $R^2$ die oben angegebene Bedeutung haben und

$R^3$ für Alkyl mit 1 bis 4 Kohlenstoffatomen steht,

in an sich bekannter Art und Weise in Gegenwart eines inerten organischen Lösungsmittels, wie beispielsweise aromatische Kohlenwasserstoffe und wie insbesondere ein Ueberschuß an eingesetztem Amidacetal bzw. Aminalester der Formeln (VIa) bzw. (VIb), in der Siedehitze umsetzt (vergleiche hierzu auch Chem.Ber. 101, 41-50(1968); J. Org.Chem.43, 4248-50(1978) sowie die Herstellungsbeispiele).

Die Amidacetale und Aminalester der Formeln (VIa) bzw. (VIb) sind allgemeine bekannte Verbindungen der organischen Chemie (vergleiche z.B. Chem.Ber.101, 41-50 (1968) und J. Org.Chem. 43, 4248-50(1978)).

Die außerdem für die erfindungsgemäße Umsetzung gemaß Verfahren (a) als Ausgangsstoffe zu verwendenden magnesium-organischen Verbindungen sind durch die Formel (III) allgemein definiert. In dieser Formel steht R vorzugsweise für diejenigen Reste, die bereits im Zusammenhang mit der Beschreibung der erfindungsgemäßen Stoffe der Formel (I) vorzugsweise für diese Substituenten genannt wurden. Hal steht vorzugsweise für Chlor oder Brom.

Die als Ausgansstoffe für das erfindungsgemäße Verfahren (b) zu verwendenden Triazolyl-ketone sind durch die Formel (IV) allgemein definiert. In dieser Formel stehen X und n vorzugsweise für diejenigen Reste, die bereits im Zusammenhang mit der Beschreibung der erfindungsgemäßen Stoffe der Formel (I) vorzugsweise für diese Substituenten genannt wurden.

Die Triazolyl-ketone der Formel (IV) sind bekannt (vergleiche z.B. DE-OS 24 31 407, DE-OS 26 10 022 und DE-OS 26 38 470; bzw. lassen sich nach üblichen Methoden herstellen, indem man die entsprechenden Halogen-ketone in Gegenwart eines Säurebinders mit 1,2,4-Triazol umsetzt.

Die außerdem für das erfindungsgemäße Verfahren (b) als Ausgangsstoffe zu verwendenden Aldehyde sind durch die Formel (V) allgemein definiert. In dieser Formel steht R vorzugsweise für diejenigen Reste, die bereits im Zusammenhang mit der Beschreibung der erfindungsgemäßen Stoffe der Formel (I) für diesen Substituenten genannt werden.

Die Aldehyde der Formel (V) sind allgemein bekannte Verbindungen der organischen Chemie.

Als Lösungsmittel kommen für die erfindungsgemäße Umsetzung gemäß Verfahren (a) vorzugsweise inerte organische Lösungsmittel in reiner Form oder im Gemisch infrage. Hierzu gehören vorzugsweise Ether, wie Diethylether, Methylethylether, Tetrahydrofuran oder Dioxan, aliphatische und aromatische Kohlenwasserstoffe, wie insbesondere Benzol, Toluol oder Xylol, sowie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man zwischen -50 und + 150°C, vorzugsweise zwischen -20 und + 120°C.

Die erfindungsgemäße Umsetzung gemäß Verfahren (a) kann in Gegenwart eines Inertgases, wie z.B. Stickstoff oder Helium, durchgeführt werden.

Bei der Durchführung des erfindungemäßen Verfahrens (a) setzt man auf 1 Mol Ketoenamin der Formel (II) vorzugsweise 1 bis 1,5 Mol an magnesium-organischer Verbindung der Formel (III) ein. Die Isolierung der Verbindungen der Formel (I) erfolgt in üblicher Weise.

Als Lösungsmittel kommen für das erfindungsgemäße Verfahren (b) vorzugsweise inerte organische Lösungsmittel infrage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Das erfindungsgemäße Verfahren (b) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen, wie Pyridin und Piperidin; sowie insbesondere Piperidinacetat.

Die Reaktionstemperaturen können bei der Durchführung des erfindungsgemäßen Verfahrens (b) in einem größeren Bereich variiert werden. Im allgemeinen arbeitet man zwischen 20 und 160°C, vorzugsweise bei der Siedetemperatur des jeweiligen Lösungsmittels.

Bei der Durchführung des erfindungsgemäßen Verfahrens(b) setzt man auf 1 Mol Triazol-keton der Formel (IV)1 bis 1,5 Mol Aldehyd der Formel (V) und katalytische bis 0,2 molare Mengen an Katalysator ein. Die Isolierung der

Verbindungen der Formel (I) erfolgt in üblicher Weise.

Zur Herstellung von physiologisch verträglichen Säureadditionssalzen der Verbindungen der Formel (I) kommen vorzugsweise folgende Säuren infrage: Die Halogenwasserstoffsäuren, wie z.B. Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure, Milchsäure, sowie Sulfonsäuren,wie z.B. p-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV.Hauptgruppe und der I. und II. sowie IV. bis VIII. Nebengruppe infrage, wobei Kupfer, Zink, Mangan, Magnesium, Zinn,Eisen und Nickel beispielhaft genannt seien.
Als Anionen der Salze kommen solche in Betracht, die sich vorzugsweise von folgenden Säuren ableiten: Halogenwasserstoffsäuren, wie z.B. die Chlorwasserstoffsäure und die Bromwasserstoffsäure, ferner Phosphorsäure, Salpetersäure und Schwefelsäure.

Die Metallkomplexe von Verbindungen der Formel (I) können in einfacher Weise nach üblichen Verfahren erhalten werden, so z.B. durch Lösen des Metallsalzes in Alkohol, z. B. Ethanol und Hinzufügen zur Verbindung der Formel (I). Man kann Metallsalz-Komplexe in bekannter Weise, z.B. durch Abfiltrieren, Isolieren und gegebenenfalls durch Umkristallisieren reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäß verwendbaren Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gersten- bzw. des Getreidemehltaus (Erysiphe graminis) und des Gurkenmehltaus (Erysiphe cichoracearum), sowie von Podosphaera-Arten, wie gegen den Erreger des Apfelmehltaus (Podosphaera leucotricha).
Besonders hervorzuheben ist, daß die erfindungsgemäßen Wirkstoffe nicht nur eine protektive Wirkung aufweisen, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthalinc, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

0040367

Herstellungsbeispiel

Beispiel 1

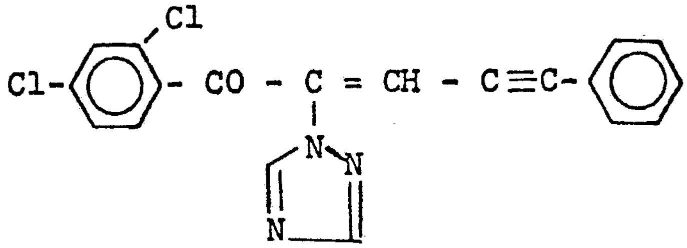

(Verfahren a)

31,2g (0,1 Mol) 1-(2,4-Dichlorphenyl)-3-dimethylamino-2-(1,2,4-triazol-1-yl)-2-propen-1-on werden in 400 ml Tetrahydrofuran gelöst. Dazu tropft man bei -10°C innerhalb von 15 Minuten eine Lösung von 26,7g (0,13 Mol) Phenylacetylen-magnesiumbromid in 70ml Tetrahydrofuran. Nach beendeter Zugabe läßt man das Reaktionsgemisch innerhalb von ca.2. Stunden auf Raumtemperatur erwärmen. Danach wird mit verdünnter Salzsäure versetzt, die organische Phase wird abgetrennt, mit Wasser gewaschen, über Natriumsulfat getrocknet und eingeengt. Man erhält 30g (81,5% der Theorie) 1-(2,4-Dichlorphenyl)-5-phenyl-2-(1,2,4-triazol-1-yl)-pent-2-en-4-in -1-on als Oel.

Herstellung des Ausgangsproduktes

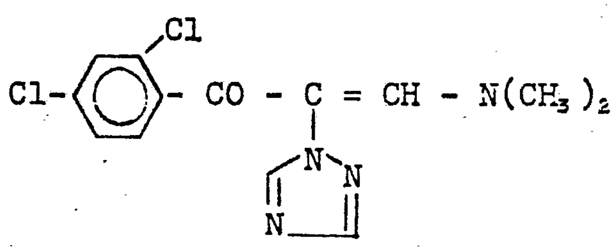

266g (1 Mol) ω-(1,2,4-Triazol-1-yl)-2,4-dichloracetophenon werden mit 131g (1,1 Mol) Dimethylformamid-dimethylacetal 5 Stunden unter Rückfluß erhitzt. Danach wird das überschüssige Acetal abdestilliert. Das zurückbleibende Oel kristallisiert beim Abkühlen. Man erhält 292g (94 % der Theorie) 1-(2,4-Dichlorphenyl)-3-dimethyl-amino-2-(1,2,4-triazol-1-yl)-prop-2-en-1-on vom Schmelzpunkt 173°C.



BAD ORIGINAL

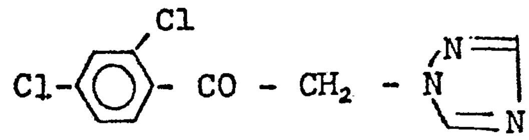

269g (1 Mol) ω-Brom-2,4-dichloracetophenon werden in 250 ml Acetonitril gelöst. Diese Lösung tropft man zu einer unter Rückfluß siedenden Suspension von 69 g 1,2,4-Triazol (1 Mol) und 150g Kaliumcarbonat in 2000 ml Acetonitril. Nach 18 bis 24 stündigem Erhitzen unter Rückfluß wird die erkaltete Suspension filtriert, das Filtrat vom Lösungsmittel befreit und der Rückstand mit Essigester aufgenommen, mit Wasser gewaschen , über Natriumsulfat getrocknet und vom Lösungsmittel befreit. Der Essigester-Rückstand kristallisiert beim Versetzen mit Isopropanol aus. Nach dem Umkristallisieren aus Ligroin/ Isopropanol erhält man 154g (60 % der Theorie) ω-(1,2,4-Triazol-1-yl)-2,4-dichloracetophenon vom Schmelzpunkt 117°C.

Beispiel 2

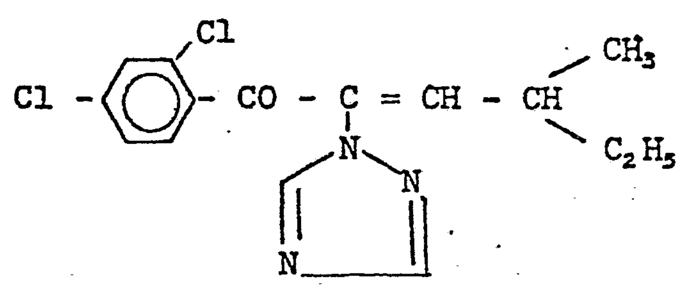

(Verfahren b)

25,6g (0,1 Mol) ω-(1,2,4-Triazol-1-yl)-2,4-dichloracetophenon und 8,6g (0,1 Mol) 2-Methyl-butyraldehyd werden zusammen mit 1 ml Piperidin und 1,5 ml Eisessig in 200 ml Toluol am Wasserabscheider unter Rückfluß erhitzt, bis die theoretische Wassermenge (1,8 ml) ausgekreist ist. Nach dem Abkühlen wird die Reaktionslösung mit gesättigter Natriumchloridlösung gewaschen, die organische Phase wird abgetrennt, über Natriumsulfat getrocknet, filtriert und eingeengt. Der ölige Rückstand wird in heißem Ligroin aufgenommen, aus dem beim Abkühlen ein farbloser kristalliner Feststoff ausfällt. Man erhält 17,2g (53,2 % der Theorie) 1-(2,4-Dichlorphenyl)-4-methyl-2-(1,2,4-triazol-1-yl)-2-hexen-1-on vom Schmelzpunkt 101-104°C.

Herstellung des Ausgangsproduktes

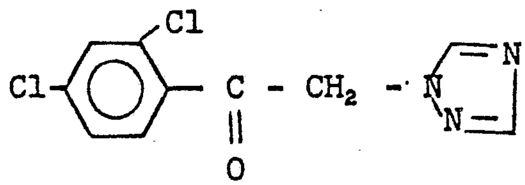

269g (1 Mol) ω-Brom-2,4-dichloracetophenon werden in 250 ml Acetonitril gelöst. Diese Lösung tropft man zu einer unter Rückfluß siedenden Suspension von 69g ( 1 Mol) 1,2,4-Triazol und 150g Kaliumcarbonat in 2000 ml Acetonitril. Nach 20-stündigem Erhitzen unter Rückfluß wird die erkaltete Suspension filtriert, das Filtrat vom Lösungsmittel befreit und der Rückstand mit Essigester aufgenommen, mit Wasser gewaschen, über Natriumsulfat getrocknet und vom Lösungsmittel befreit. Der Essigester-Rückstand kristallisiert beim Versetzen mit Isopropanol aus. Nach dem Umkristallisieren aus Ligroin/Isopropanol erhält man 154g (60 % der Theorie) ω-(1,24- Triazol-1-yl)-2,4-dichloracetophenon vom Schmelzpunkt 117°C.

Analog werden die folgenden Verbindungen der allgemeinen Formel (I)

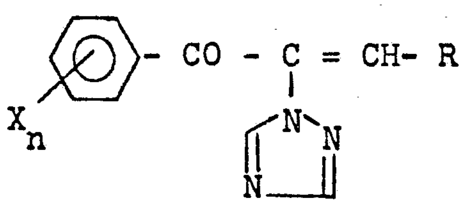 (I)

erhalten:

| Bsp. Nr. | $X_n$ | R | Schmelzpunkt(°C) Brechungsindex $n_D^{20}$ |
|---|---|---|---|
| 3 | 2,4-$Cl_2$ | $CH_3$ | 105-07 |
| 4 | 2,4-$Cl_2$ | $C_2H_5$ | Oel |
| 5 | 2,4-$Cl_2$ | i-$C_3H_7$ | 63-67 |
| 6 | 2,4-$Cl_2$ | $C(CH_3)_3$ | 155-57 |
| 7 | 2,4-$Cl_2$ | n-$C_7H_{15}$ | 1,5320 |
| 8 | 2,4-$Cl_2$ | -CH(CN)- 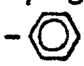 -Cl | 180-85 |
| 9 | 4-Br | $CH_3$ | Oel |
| 10 | 4-Br | $C_2H_5$ | Oel |
| 11 | 4-Br | n-$C_4H_9$ | Oel |
| 12 | 4-O- 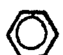 -Cl | $C_2H_5$ | 1,6080 |
| 13 | 4-O- 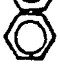 -Cl | $CH_3$ | 1,6188 |
| 14 | 4-Cl | $-CH_2-$ 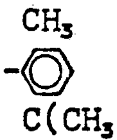 | 1,6112 |
| 15 | 2,Cl;4-$C(CH_3)_3$ | $C(CH_3)_3$ | Oel |
| 16 | 4-Br | n-$C_7H_{15}$ | Oel |
| 17 | 2,4-$Cl_2$ | $-CH_2-C(CH_3)=CH_2$ | 1,5724 |
| 18 | 4-Cl | $CH_3$ | 1,5987 |
| 19 | 4-Cl | -C≡C- 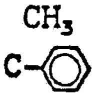 | 120-23 |
| 20 | 4-Cl | $C_2H_5$ | 1,5812 |
| 21 | 2,4-$Cl_2$ | n-$C_4H_9$ | 1,5672 |
| 22 | 2,4-$Cl_2$ | n-$C_4H_9$ | 101-05 (x$CuCl_2$) |
| 23 | 2,4-$Cl_2$ | n-$C_7H_{15}$ | Oel(x$CuCl_2$) |
| 24 | 2,4-$Cl_2$ | i-$C_3H_7$ | 96-99 (x$CuCl_2$) |
| 25 | 2,4-$Cl_2$ | $CH_3$ | 98-102 (x$CuCl_2$) |
| 26 | 2,4-$Cl_2$ | $C_2H_5$ | 83(x$CuCl_2$) |
| 27 | 4-Br | n-$C_7H_{15}$ | Oel(x$CuCl_2$) |
| 28 | 4-Br | $C_2H_5$ | 82(x$CuCl_2$) |
| 29 | 4-O- 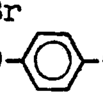 -Cl | $CH_3$ | 85-90 (x$CuCl_2$) |
| 30 | 4-Cl | $-CH_2-$ 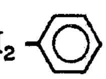 | 130-33 (x$CuCl_2$) |
| 31 | 4-Cl | $C_2H_5$ | 92-94 (x$CuCl_2$) |
| 32 | 2,4-$Cl_2$ | $-CH(CH_3)-C_2H_5$ | 88-92(x$CuCl_2$) |

| Bsp. Nr. | $X_n$ | R | Schmelzpunkt(°C) Brechungsindex($n_D^{20}$) |
|---|---|---|---|
| 33 | 2,4-$Cl_2$ | 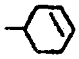 | 105 |
| 34 | 2,4-$Cl_2$ | $-CH(C_2H_5)-C_4H_9-n$ | 73 |
| 35 | 2,4-$Cl_2$ | $-CH(C_2H_5)_2$ | 88 |
| 36 | 2,4-$Cl_2$ | - 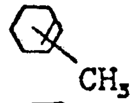 | Oel |
| 37 | 2,4-$Cl_2$ | - 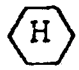 | Oel |
| 38 | 2,4-$Cl_2$ | $-CH(CH_3)-C_3H_7-n$ | Oel |
| 39 | 4-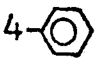 | $-CH(C_2H_5)-C_4H_9-n$ | Oel |
| 40 | 4-F | $-CH(CH_3)-C_2H_5$ | Oel |
| 41 | 4-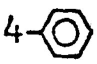 | $-CH(CH_3)-C_2H_5$ | Oel |
| 42 | 4-F | $-CH(C_2H_5)-C_4H_9-n$ | Oel |
| 43 | 4-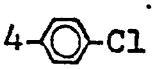 | $-CH(CH_3)_2$ | 84 |
| 44 | 4-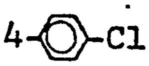 | $-CH(C_2H_5)-C_4H_9-n$ | Oel |
| 45 | 4-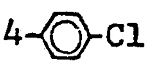 | 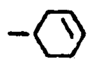 | Oel |
| 46 | 4-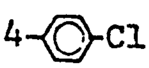 | $-CH(C_2H_5)_2$ | Oel |
| 47 | 2,4-$Cl_2$ | 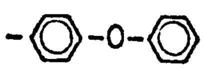 | Oel |

## Anwendungsbeispiele

In den nachfolgenden Beispielen werden die nachstehend angegebenen Verbindungen als Vergleichssubstanzen eingesetzt:

(A) 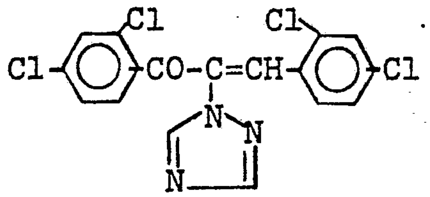

(B) 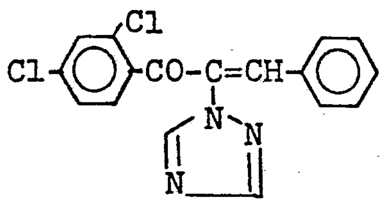

(C) 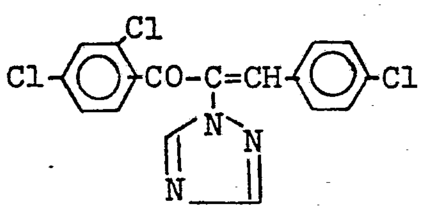

(D) 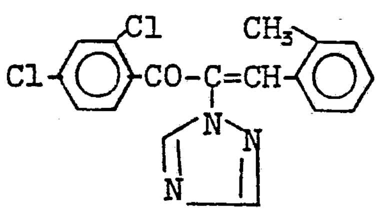

(E) 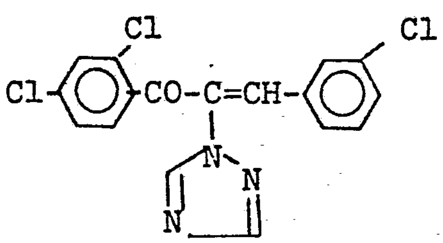

0040367

Beispiel A

Erysiphe-Test (Gerste) /protektiv/

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.
Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele: 3,6,5,32,25,24,34,35, 2 ,43 und 46.

Tabelle A

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 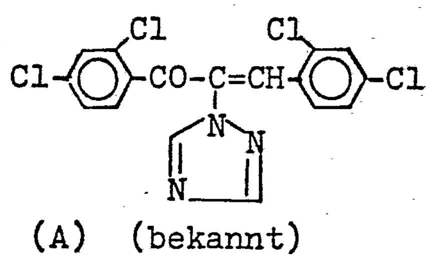 (A) (bekannt) | 0,0025 | 75,0 |
| 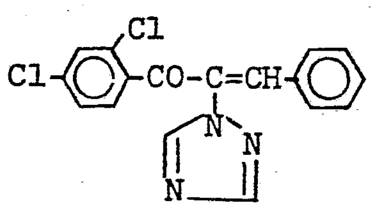 (B) ( bekannt) | 0,0025 | 83.8 |
| 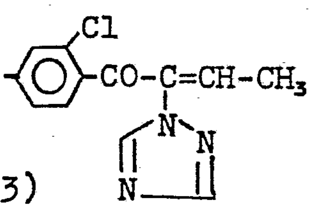 (3) | 0,0025 | 33,8 |
| 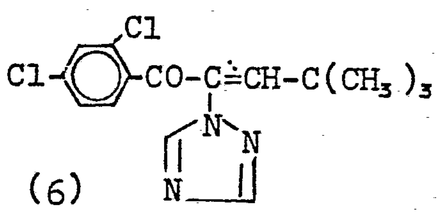 (6) | 0,0025 | 0,0 |
| 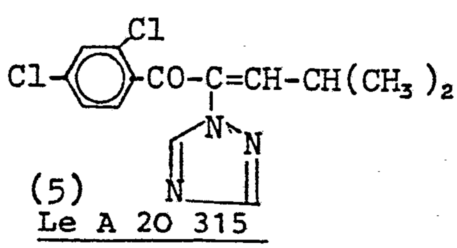 (5) | 0,0025 | 12,5 |

Tabelle A (Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 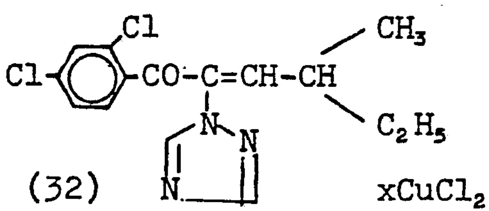 (32) $xCuCl_2$ | 0,0025 | 0,0 |
| 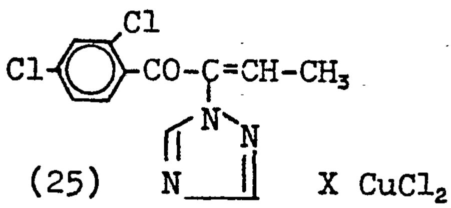 (25) X $CuCl_2$ | 0,0025 | 33,8 |
| 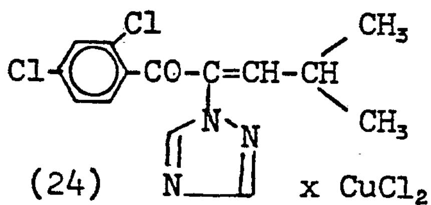 (24) x $CuCl_2$ | 0,0025 | 13,8 |
| 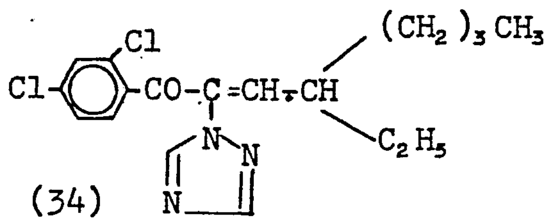 (34) | 0,0025 | 25,0 |
| 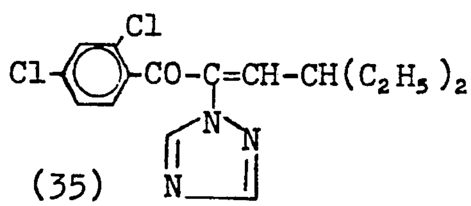 (35) | 0,0025 | 0,0 |

Tabelle A (Fortsetzung)

Erysiphe-Test (Gerste) / protektiv

| Wirkstoff | Wirkstoffkonzentration in der Spritzbrühe in Gew.-% | Krankheitsbefall in % der unbehandelten Kontrolle |
|---|---|---|
| 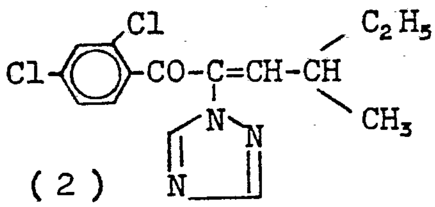 (2) | 0,0025 | 21,3 |
| 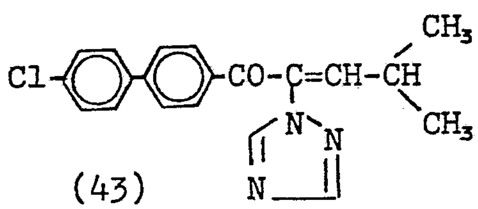 (43) | 0,0025 | 25,0 |
| 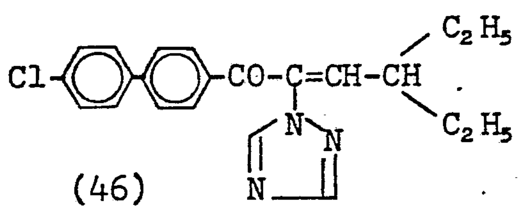 (46) | 0,0025 | 33,8 |

Beispiel B

Gerstenmehltau-Test (Erysiphe graminis var.hordei)/ systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var.hordei bestäubt und bei 21-22°C und 80-90% rel.Luftfeuchte und 16-stündiger Belichtung weiter kultiviert. Innerhalb von 6 Tagen bilden sich an den Blättern die typischen Mehltaupusteln aus.
Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.
Eine deutliche Überlegenheit in der Wirksamkeit gegenüber dem Stand der Technik zeigen bei diesem Test z.B. die Verbindungen gemäß folgender Herstellungsbeispiele: 5, 32 , 35 und 2.

## Tabelle B

Gerstenmehltau-Test (Erysiphe graminis var.hordei)/
systemisch

| Wirkstoffe | Wirkstoffkonzentration im Beizmittel in Gew.% | Beizmittelaufwandmenge in g/kg Saatgut | Befall in % der unbehandelten Kontrolle |
|---|---|---|---|
| 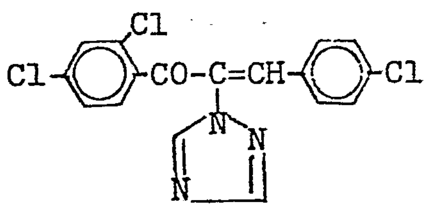 (C) (bekannt) | 25 | 10 | 100 |
| 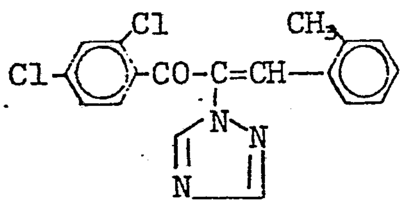 (D) (bekannt) | 25 | 10 | 100 |
| 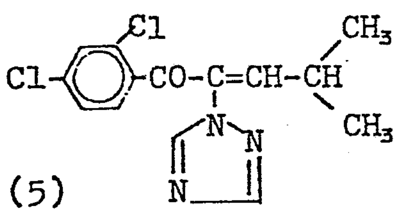 (5) | 25 | 4 | 0,0 |
| 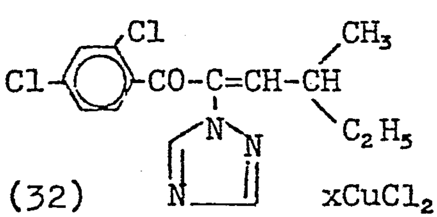 (32) xCuCl$_2$ | 25 | 4 | 0,0 |
| 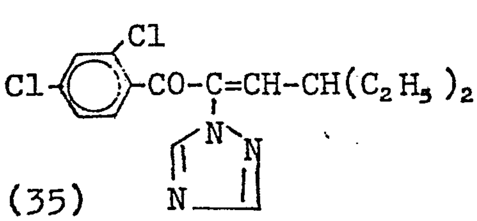 (35) | 25 | 10 | 16,3 |

Tabelle B (Fortsetzung)

Gerstenmehltau-Test (Erysiphe graminis var.hordei)/ systemisch

| Wirkstoffe | Wirkstoff-konzentration im Beizmittel in Gew.% | Beizmittel-aufwandmenge in g/kg Saatgut | Befall in % der unbehandelten Kontrolle |
|---|---|---|---|
| 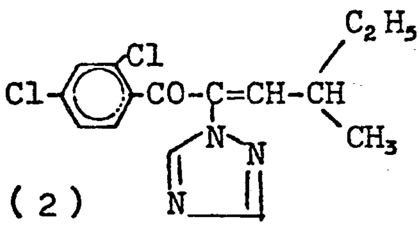 (2) | 25 | 10 | 0,0 |

Beispiel C

Erysiphe-Test (Gurken)/ Protektiv

Lösungsmittel: 4,7 Gewichtsteile Aceton

Emulgator: 0,3 Gewichtsteile Alkyl-aryl-polyglykolether

Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 Stunden im Gewächshaus. Dann werden sie zur Inokulation mit Konidien des Pilzes Erysiphe cichoracearum bestäubt. Die Pflanzen werden anschließend bei 23 bis 24°C und bei einer relativen Luftfeuchtigkeit von ca 75 % im Gewächshaus aufgestellt.

Nach 12 Tagen wird der Befall der Gurkenpflanzen bestimmt. Die erhaltenen Boniturwerte werden in Prozent Befall umgerechnet. 0 % bedeutet keinen Befall, 100 % bedeutet, daß die Pflanzen vollständig befallen sind.

Eine deutliche Ueberlegenheit in der Wirksamkeit gegenüber dem Stand der Technik zeigen bei diesem Test z.B. die Verbindungen gemäß folgender Herstellungsbeispiele: 6, 32 und 35.

## Tabelle C

Erysiphe-Test (Gurken) / Protektiv

| Wirkstoff | Befall in % bei einer Wirkstoffkonzentration von 0,0005 % |
|---|---|
| 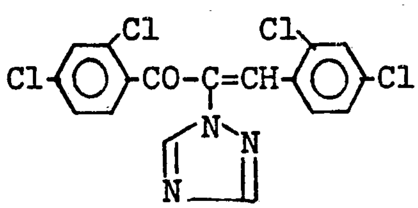 (A) (bekannt) | 37 |
| 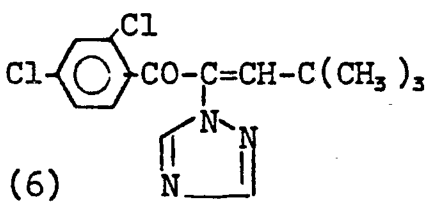 (6) | 12 |
| 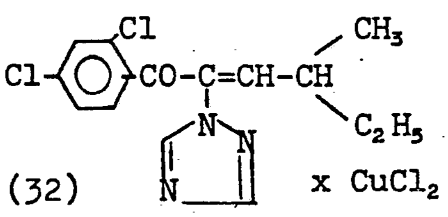 (32) | 12 |
| 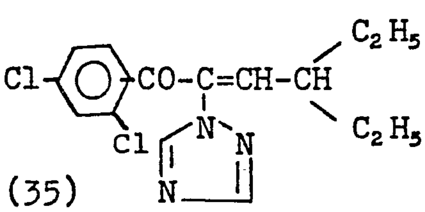 (35) | 16 |

Beispiel D

Podosphaera-Test (Apfel) / protektiv

Lösungsmittel: 4,7 Gewichtsteile Aceton

Emulgator : 0,3 Gewichtsteile Alkyl-aryl-polyglkolether

Wasser : 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.
Mit der Spritzflüssigkeit bespritzt man junge Apfelsämlinge, die sich im 4- bis 5-Blattstadium befinden, bis zur Tropfnässe. Die Pflanzen verbleiben 24 Stunden bei 20°C und einer relativen Luftfeuchtigkeit von 70% im Gewächshaus. Anschließend werden die durch Bestäuben mit Konidien des Apfelmehltauerregers (Podosphaera leucotricha) inokuliert und in ein Gewächshaus mit einer Temperatur von 21 bis 23 °C und einer relativen Luftfeuchtigkeit von ca.70 ° gebracht.
10 Tage nach der Inokulation wird der Befall der Sämlinge bestimmt. Die erhaltenen Boniturwerte werden in Prozent Befall umgerechnet. 0 % bedeutet keinen Befall, 100 % bedeutet, daß die Pflanzen vollständig befallen sind.

Eine deutliche Ueberlegenheit in der Wirlsamkeit gegenüber dem Stand der Technik zeigen bei diesem Test z.B. die Verbindungen gemäß folgender Herstellungsbeispiele: 6,1,5,32,25,34, 2 , 37,38,40,43,44 und 46.

## T a b e l l e D

Podosphaera - Test (Apfel)/ protektiv

| W i r k s t o f f | Befall in % bei einer Wirkstoffkonzentration von 0,001 % |
|---|---|
| 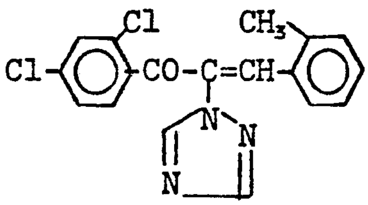 (D) (bekannt) | 76 |
| 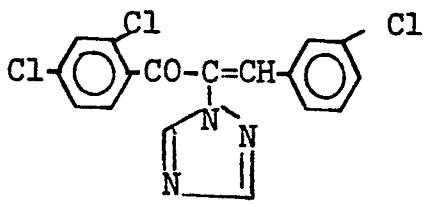 (E) (bekannt) | 37 |
| 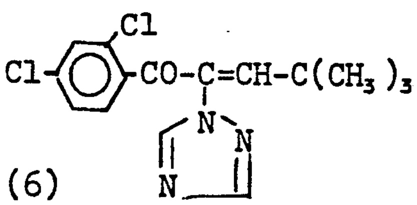 (6) | 0 |
| 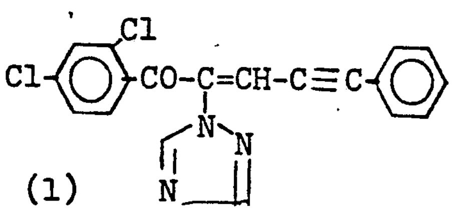 (1) | 30 |
| 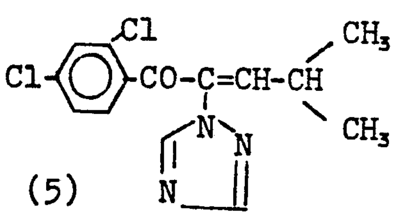 (5) | 1 |

## Tabelle D (Fortsetzung)

Podosphaera - Test (Apfel)/ protektiv

| Wirkstoff | Befall in % bei einer Wirkstoffkonzentration von 0,001 % |
|---|---|
| 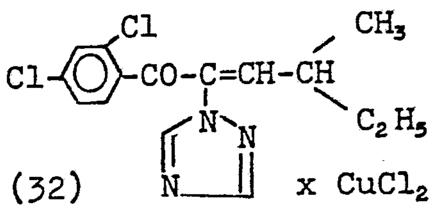 (32) x $CuCl_2$ | 0 |
| 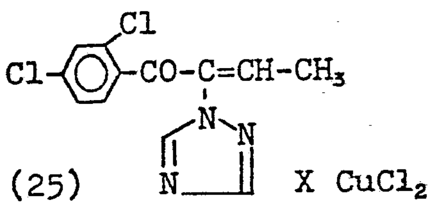 (25) X $CuCl_2$ | 17 |
| 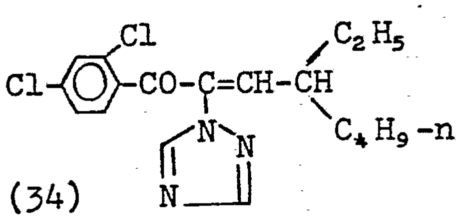 (34) | 7 |
| 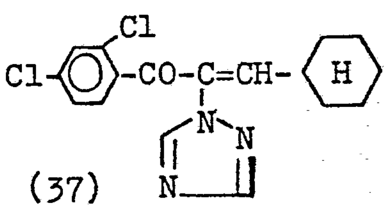 (37) | 24 |
| 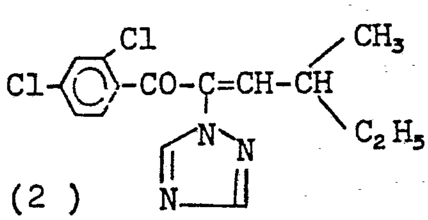 (2 ) | 1 |

Tabelle D (Fortsetzung)

Podosphaera - Test (Apfel)/ protektiv

| Wirkstoff | Befall in % bei einer Wirkstoffkonzentration von 0,001 % |
|---|---|
| 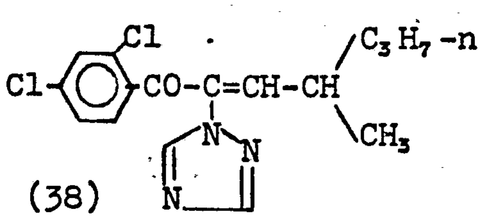 (38) | 1 |
| 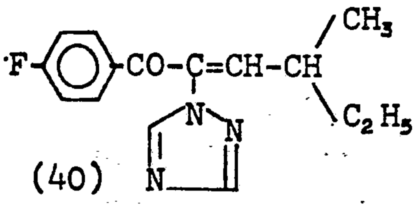 (40) | 22 |
| 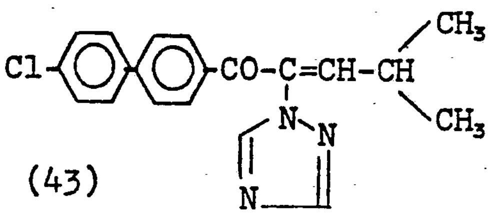 (43) | 16 |
| 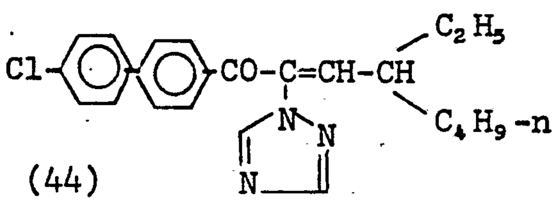 (44) | 15 |
| 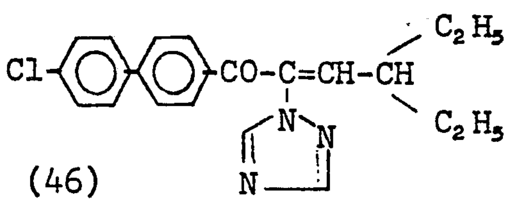 (46) | 5 |

Patentansprüche

1) Substituierte Phenyl-triazolyl-vinyl-ketone der allgemeinen Formel

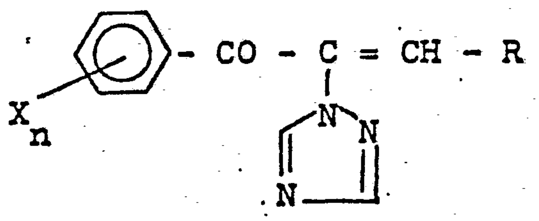

(I)

in welcher

R für Methyl, Ethyl, Isopropyl, Alkyl mit mehr als 3 Kohlenstoffatomen, gegebenenfalls substituiertes Cycloalkyl und Cycloalkenyl, gegebenenfalls substituiertes Alkenyl und Alkinyl, gegebenenfalls substituiertes Phenoxyphenyl sowie gegebenenfalls im Alkylteil und im Phenylteil substituiertes Phenylalkyl steht,

X für Halogen, gegebenenfalls substituiertes Phenyl und Phenoxy steht und

n für die Zahlen 0, 1, 2 und 3 steht,

und deren physiologisch verträglichen Säureadditions-Salze sowie Metallsalz-Komplexe.

2) Verfahren zur Herstellung von substituierten Phenyl-triazolyl-vinyl-ketonen der allgemeinen Formel I in Anspruch 1, dadurch gekennzeichnet, daß man

a) Ketoenamine der Formel

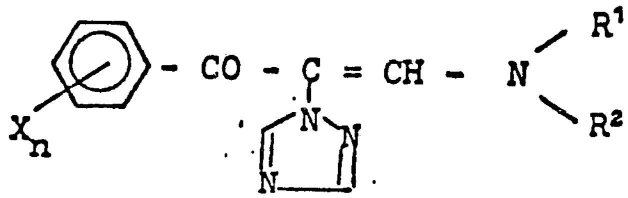 (II)

in welcher

X und n die oben angegebene Bedeutung haben und
$R^1$ und $R^2$ gleich oder verschieden sind und für Alkyl stehen,

mit magnesium-organischen Verbindungen der Formel

$$Hal - Mg - R \qquad (III)$$

in welcher

R die oben angegebene Bedeutung hat und
Hal für Halogen steht,

in Gegenwart eines Lösungsmittels und gegebenenfalls in Gegenwart eines Katalysators umsetzt, oder

b) Triazolyl-ketone der Formel

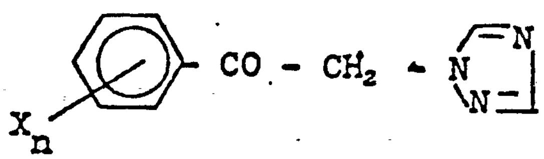 (IV)

in welcher

X und n die oben angegebene Bedeutung haben,

mit Aldehyden der Formel

$$O = CH - R \qquad (V)$$

in welcher

R die oben angegebene Bedeutung hat,

in Gegenwart eines Lösungsmittels und in Gegenwart eines Katalysators umsetzt, und noch gegebenenfalls an die so erhaltenen Verbindungen eine Säure oder ein Metallsalz addiert.

3) Fungizide Mittel, gekennzeichnet durch einen Gehalt an mindestens einem substituierten Phenyl-triazolyl-vinyl-keton der Formel I.

4) Verfahren zur Bekämpfung von Pilzen, dadurch gekennzeichnet, daß man substituierte Phenyl-triazolyl-vinyl-ketone der Formel I auf Pilze oder ihren Lebensraum einwirken läßt.

5) Verwendung von substituierten Phenyl-triazolyl-vinyl-ketonen der Formel I zur Bekämpfung von Pilzen.

6) Verfahren zur Herstellung von fungiziden Mitteln, dadurch gekennzeichnet, daß man substituierte Phenyl-triazolyl-vinyl-ketone der Formel I mit Streckmitteln und/oder oberflächenaktiven Mitteln vermischt.

0040367

Europäisches Patentamt

# EUROPÄISCHER RECHERCHENBERICHT

Nummer der Anmeldung
EP 81 10 3478

| EINSCHLÄGIGE DOKUMENTE | | | KLASSIFIKATION DER ANMELDUNG (Int. Cl.[3]) |
|---|---|---|---|
| Kategorie | Kennzeichnung des Dokuments mit Angabe, soweit erforderlich, der maßgeblichen Teile | betrifft Anspruch | |
| | US - A - 4 182 862 (HAK-FOON CHAN)<br>* Patentschrift *<br>-- | 1 | C 07 D 249/08<br>A 01 N 43/64 |
| D | DE - A - 2 645 617 (I.C.I.)<br>* Ansprüche *<br>-- | 1-6 | |
| P | EP - A - 0 015 387 (BAYER)<br>* Ansprüche *<br>-- | 1-6 | RECHERCHIERTE SACHGEBIETE (Int. Cl.[3]) |
| AP | EP - A - 0 024 538 (BAYER)<br>---- | 1-6 | C 07 D 249/08 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 07-08-1981 | CREMERS |